Europäisches Patentamt

European Patent Office

Office européen des brevets

(19)

(11) **EP 0 524 083 B1**

(12) ## FASCICULE DE BREVET EUROPEEN

(45) Date de publication et mention
de la délivrance du brevet:
**06.03.1996 Bulletin 1996/10**

(51) Int. Cl.⁶: **A61B 5/00**

(21) Numéro de dépôt: **92402032.4**

(22) Date de dépôt: **15.07.1992**

(54) **Procédé non invasif de détermination in vivo du taux de saturation en oxygène du sang artériel, et dispositif mettant en oeuvre le procédé**

Nichtinvasives Verfahren zur in vivo Bestimmung, der arteriellen Blutsauerstoffsättigungsrate und Vorrichtung dafür

Non-invasive method for determining, in vivo, the rate of oxygen saturation of arterial blood and device for carrying out this method

(84) Etats contractants désignés:
**AT BE CH DE DK ES GB GR IT LI LU MC NL PT SE**

(30) Priorité: **17.07.1991 FR 9109020**

(43) Date de publication de la demande:
**20.01.1993 Bulletin 1993/03**

(73) Titulaire: **EFFETS BIOLOGIQUES DE L'EXERCICE dit GIP EXERCICE,
(Groupement d'interêt publique)
F-42000 Saint-Etienne (FR)**

(72) Inventeurs:
• **Barthelemy, Jean Claude
F-42000 Saint Etienne (FR)**
• **Geyssant, André
F-421000 Saint Etienne (FR)**

(74) Mandataire: **CABINET BONNET-THIRION
95 Boulevard Beaumarchais
F-75003 Paris (FR)**

(56) Documents cités:
| | |
|---|---|
| **EP-A- 0 380 664** | **EP-A- 0 426 358** |
| **WO-A-82/01948** | **WO-A-90/04941** |
| **US-A- 4 714 341** | **US-A- 4 819 752** |

## Description

L'invention se rapporte à un procédé de détermination du taux de saturation en oxygène du sang artériel d'un sujet, par comparaison du taux d'oxyhémoglobine, ou $HbO_2$ au taux d'hémoglobine total, où l'on acquiert, sur un capteur opto-électronique des signaux représentatifs d'absorption optique sur deux trajets, traversant une région tissulaire appropriée du sujet et émanant de deux sources lumineuses respectives émettant chacune dans une bande de longueur d'onde, l'une dans le proche infrarouge et l'autre dans le rouge profond, en sorte que ces signaux représentatifs comportent une composante variable correspondant à l'absorption par le sang artériel pulsatile, et une composante continue correspondant à l'absorption par les autres tissus traversés, on sépare la composante variable de chaque signal représentatif, on calcule, suivant des formules en soi connues, à partir des composantes variables, les taux respectifs $HbO_2$ et en déoxyhémoglobine, ou Hb, et on en déduit, de façon également connue, le taux de saturation en oxygène.

L'invention se rapporte également à un dispositif pour la mise en oeuvre du procédé de l'invention.

La détermination du taux de saturation en oxygène du sang artériel permet d'atteindre la capacité de l'organisme d'un sujet à répondre aux appels en oxygène des différents organes, et par suite, d'apprécier soit les limites d'activité physiques tolérables sans danger, soit l'opportunité d'une oxygénothérapie.

De façon courante, la détermination du taux de saturation en oxygène du sang artériel, ou $SaO_2$, est faite par prélèvement de sang artériel, et analyse de ce sang. Il s'agit de déterminations à un instant donné, qui ne donnent pas d'indications sur les variations dans le temps en réponse à des sollicitations organiques.

Entre les années 1930 et 1950 ont été conçus des oxymètres notamment pour surveiller les hypoxies sévères auxquelles étaient soumis les pilotes (Nicolai 1932, Matthes 1935, Squire 1940, Millikan 1942, Wood et Geraci 1949) avec lesquels on opérait par mesure de l'absorption optique par des tissus, en déduisant d'une absorption globale l'absorption des tissus rendus exsangues par compression à l'aide d'un ballonnet gonflable.

Aoyagi (1974) a développé un procédé de mesure de la saturation artérielle en continu, dit oxymétrie de pouls. Ce procédé est fondé sur les considérations suivantes :

- les tissus mous sont relativement transparents dans le rouge profond et le proche infrarouge ;
- par contre l'hémoglobine, sous ses différentes formes incluant l'oxyhémoglobine, et là déoxyhémoglobine présentent des absorptions marquées dans ces parties du spectre ;
- l'oxyhémoglobine présente une courbe d'absorption en fonction de la longueur d'onde qui est, par rapport à la courbe d'absorption de la déoxyhémoglobine, inférieure dans le rouge profond, et supérieure dans le proche infrarouge, de sorte que, à partir de deux mesures à des longueurs d'ondes, l'une dans le proche infrarouge, l'autre dans le rouge profond, connaissant les coefficients spécifiques d'absorption des deux formes d'hémoglobine, on peut calculer les teneurs relatives en ces deux formes, dans la mesure où seules interviennent les absorptions des formes d'hémoglobine ;
- dans le réseau artériel, le débit de sang est pulsé, au rythme cardiaque ; dans une région tissulaire traversée par le trajet optique entre une source lumineuse et un capteur opto-électronique, le volume de sang artériel varie au rythme du pouls, et donc l'absorption du sang artériel ; par contre l'absorption par les tissus, dans la région tissulaire précédente, autres que le sang artériel ne varie pas, que ce soient les tissus mous immobiles, et le sang veineux à écoulement constant. En conséquence, le signal issu du capteur opto-électronique va comporter une composante continue, correspondant à l'absorption par les tissus autres que le sang artériel, et une composante variable au rythme de la pulsation sanguine, qui ne dépend que de l'absorption sanguine.
- Strictement parlant, il existe un autre composant du sang qui présente des absorptions marquées dans la région qui va de l'infrarouge proche au rouge profond, à savoir la cytochrome C oxydase. Mais ce composant présente lui aussi une absorption variable avec le taux d'oxygène dans le sang, et se trouve à des teneurs faibles devant la teneur en hémoglobine, de sorte que, en négligeant d'introduire des corrections pour tenir compte de l'absorption propre de la cytochrome C oxydase, les erreurs sur la saturation en oxygène restent du second ordre.

Le procédé d'Aoyagi a été amélioré par Nakajima et al. (1975), par Yoshiya et al. (1980), Shimada et al. (1984), notamment grâce aux progrès réalisés en opto-électronique, et électronique de traitement de signal.

Payne JP et Severinghaus JW (1985) ont publié des travaux sur les courbes d'absorption des'formes d'hémoglobine qui préconisaient l'emploi de deux longueurs d'ondes, à savoir 940 nm dans le proche infrarouge, et 660 nm dans le rouge profond.

Pour le calcul de la saturation en oxygène, qui sera notée $SaO_2$, on fait intervenir les considérations suivantes :

L'absorption, exprimée par la densité optique DO, suit la loi de Beer-Lambert,

$$DO = e . \underline{c} . \underline{l} \qquad\qquad (1)$$

où e est le coefficient d'extinction moléculaire, $\underline{c}$ la concentration du composant absorbant, et $\underline{l}$ la longueur du trajet optique dans le milieu absorbant.

Or la densité optique globale est la somme des densités optiques dues aux absorptions propres des différents absorbants.

En opérant à deux longueurs d'onde, on obtient un système d'équation du premier degré à deux inconnues, qui sont les taux en oxyhémoglobine et en déoxyhémoglobine, soit $HbO_2$ et $Hb$, respectivement

$$DO_{660\ nm} = \left[ e_1(Hb) + e_2(HbO_2) \right]\ \underline{1} \quad \Bigg\}$$

$$DO_{940\ nm} = \left[ e_3(Hb) + e_4(HbO_2) \right]\ \underline{1} \quad \Bigg\} \qquad (2)$$

où $e_1$, et $e_3$ sont les coefficients d'absorption moléculaire de la déoxyhémoglobine, et $e_2$ et $e_4$ ceux de l'oxyhémoglobine, respectivement à 660 et 940 nm.

La résolution du sytème (2) est classique, et donne $Hb$ et $HbO_2$, à un facteur $\underline{l}$ près ; bien entendu, les trajets dans les tissus seront égaux pour l'une et l'autre longueurs d'onde, ce qui suppose que ces trajets soient très proches.

La saturation en oxygène $SaO_2$ est alors donnée par :

$$SaO_2 = HbO_2/(Hb + HbO_2) \qquad (3)$$

où le facteur $\underline{l}$ disparaît.

On aura compris que les valeurs de DO considérées ci-dessus correspondent à la composante variable du signal significatif de l'absorption, en raison du caractère linéaire de la combinaison des densités optiques.

Jusqu'ici, les oxymètres de pouls étaient des appareils relativement lourds et encombrants, qui n'étaient mis en oeuvre qu'en milieu hospitalier, où l'on ne pouvait effectuer des mesures de saturation en oxygène du sang artériel en continu dans des conditions proches de celles qu'impose la vie quotidienne. On était ainsi privé d'enseignements précieux sur les risques d'hypoxie sous les aspects de diagnostic, de traitement et de régime de vie.

Le document US-A-4 819 752 décrit un oxymètre de pouls de ce genre, prévu pour la surveillance de patients en salle d'opération. En raison des possibilités que les pulsations artérielles soient très faibles, l'accent est mis sur la sensibilité. Pour assurer des gains éventuellement très élevés qui restent cohérents, les signaux correspondant à l'absorption dans le rouge et l'infrarouge sont traités en alternance sur une voie unique, après un filtrage spécifique à chaque longueur d'onde. Pour le contrôle du gain de la voie, les signaux gardent une composante continue. Dans une disposition préférée, on utilise deux diodes émettrices rouges de part et d'autre de la diode infrarouge, qui sont excitées simultanément, de façon que les trajets rouge et infrarouge dans le tissu vascularisé présentent la même symétrie et soient pratiquement équivalents.

Par ailleurs, les oxymétries ne donnaient de mesures que sur $Hb$ et $HbO_2$, et négligeaient une troisième forme d'hémoglobine, la carboxyhémoglobine, qui résulte de la fixation de monoxyde de carbone sur l'hémoglobine, avec une énergie de liaison relativement forte.

Certes, pendant longtemps, sauf lorsqu'il s'agissait d'intoxication grave à l'oxyde de carbone, on a négligé l'erreur introduite par la présence de carboxyhémoglobine, ou $HbCO$, sur la mesure de la saturation en oxygène. Mais, avec le développement de la lutte contre le tabagisme et contre la pollution de l'atmosphère par les véhicules automobiles, la possibilité d'atteindre le taux $HbCO$ est apparue de plus en plus souhaitable.

Les Demandeurs se sont fixé pour but de réaliser un appareil capable de déterminer les parts respectives de $Hb$, $HbO_2$ et $HbCO$ dans le sang artériel, qui soit aisément portable, de façon à pouvoir déterminer ces taux $Hb$, $HbO_2$ et $HbCO$ sur un sujet, sur une période prolongée, par exemple de l'ordre de 24 heures, le sujet ayant une activité proche de la normale.

Dans ce but, l'invention propose un procédé de détermination sur une période prolongée, du taux de saturation en oxygène du sang artériel d'un sujet, par comparaison du taux d'oxyhémoglobine, ou $HbO_2$ au taux d'hémoglobine total, où l'on acquiert, sur un capteur optoélectronique unique, des signaux représentatifs d'absorption optique sur des trajets traversant une région tissulaire appropriée du sujet et émanant de sources lumineuses émettant alternativement dans deux bandes de longueurs d'onde, une dans le proche infrarouge et l'autre dans le rouge profond, en sorte que ces signaux représentatifs comportent une composante variable correspondant à l'absorption par le sang artériel pulsatile, et une composante continue correspondant à l'absorption par les autres tissus, on calcule, à partir des composantes variables, les taux respectifs $HbO_2$ et en déoxyhémoglobine, ou $Hb$, et on en déduit le taux de saturation en oxygène, procédé caractérisé en ce que, pour tenir compte d'un taux en carboxyhémoglobine, ou $HbCO$, on met en oeuvre une source émettant dans une bande de longueurs d'onde comprise entre les deux premières bandes citées, les trajets optiques, émanant des trois sources émettant chacune sur une differente bande de longueur d'onde parmi les trois bandes de longueur d'onde citées, étant définis en sorte d'être, dans la région tissulaire traversée, sensiblement parallèles et accolés, et aboutissant au capteur unique, on excite, en succession cyclique, les trois sources, la fréquence de succession cyclique étant grande devant la fréquence de pulsation artérielle du sujet, en sorte que les signaux représentatifs soient entrelacés, on aiguille, au rythme de la succession cyclique, chaque signal représentatif sur une voie séparée respective où l'on exprime numériquement le signal représentatif, on sépare la composante variable respective de chaque signal représentatif, et on calcule $HbO_2$, Hb et HbCO en résolvant l'équation suivante:

$$DO\lambda1 = [e_1(Hb) + e_2(HbO_2) + e_5(HbCO)] *\underline{l}$$

$$DO\lambda3 = [e_3(Hb) + e_4(HbO_2) + e_6(HbCO)] *\underline{l}$$

$$DO\lambda2 = [e_7(Hb) + e_8(HbO_2) + e_9(HbCO)] *\underline{l}$$

où $DO\lambda_n$ représente la composante variable d'absorption à la longueur d'onde $\lambda_n$, $\underline{l}$ est la profondeur de tissu traversé, et $e_j$ un coefficient d'absorption spécifique de la longueur d'onde et de l'espèce d'hémoglobine déterminé expérimentalement;

et l'on en déduit le taux de saturation en oxygène.

Il va de soi que les trajets optiques seront pris, comme il est courant, sur des épaisseurs relativement faibles de tissus bien vascularisés, par exemple sur l'oreille, ou sur les doigts.

En opérant à trois longueurs d'ondes $\lambda1$, $\lambda2$ et $\lambda3$, on a un système d'équations linéaires à trois inconnues :

$$\left.\begin{array}{l}DO\lambda1 = [e_1(Hb) + e_2(HbO_2) + e_5(HbCO)]\ \underline{l} \\ DO\lambda3 = [e_3(Hb) + e_4(HbO_2) + e_6(HbCO)]\ \underline{l} \\ DO\lambda2 = [e_7(Hb) + e_8(HbO_2) + e_9(HbCO)]\ \underline{l}\end{array}\right\} \qquad (4)$$

qui peut être résolu par des formules connues.

De façon comparable à la formule (3), la saturation en oxygène de l'hémoglobine fonctionnelle, ou $SbO_2$, sera donnée par :

$$SbO_2 = HbO_2/(Hb + HbO_2) \qquad (5)$$

la saturation en oxygène de l'hémoglobine totale, ou $SaO_2$, sera donnée par :

$$SaO_2 = HbO_2/(Hb + HbO_2 + HbCO) \qquad (6)$$

et la teneur relative en carboxyhémoglobine dans l'hémoglobine totale, ou FCO, sera donnée par :

$$FCO = HbCO/(Hb + HbO_2 + HbCO) \qquad (7)$$

Par ailleurs, en utilisant un capteur unique, on favorisera la concentration des trajets optiques afin d'opérer sur des tissus aussi identiques que possible, en réduisant les inhomogénéités des tissus traversés, et les différences de longueur. En excitant cycliquement les trois sources lumineuses en régime d'impulsion, à une fréquence grande devant la fréquence de pulsation artérielle, on reconstitue, après l'aiguillage sur les voies respectives, les courbes dépendantes de la pulsation, dont on peut séparer les composantes variables.

De préférence, les bandes de longueurs d'onde sont centrées sur 940 nm, 660 nm et 750 nm, respectivement.

En adoptant une longueur d'onde intermédiaire pour le troisième chemin optique, on obtient un pouvoir séparateur satisfaisant, grâce aux écarts entre absorptions spécifiques pour les trois espèces d'hémoglobine ; et on n'introduit pas de discontinuités d'absorption pour les tissus non sanguins.

De préférence, le traitement des signaux représentatifs d'absorption, comprenant l'extraction des composantes variables et les calculs subséquents sont effectués dans un microprocesseur sous la commande d'un logiciel approprié.

Sous un autre aspect, l'invention propose un dispositif pour la détermination, sur une période prolongée, du taux de saturation en oxygène du sang artériel d'un sujet, par comparaison du taux en oxyhémoglobine, ou $HbO_2$, au taux d'hémoglobine total, comportant des moyens aptes à définir des trajets optiques traversant une région tissulaire appropriée du sujet entre des sources lumineuses émettant alternativement dans deux bandes de longueurs d'onde différentes situées respectivement dans l'infrarouge proche et dans le rouge profond, et un capteur optoélectronique unique apte à émettre des signaux représentatifs de l'absorption optique sur les trajets et comportant une composante variable correspondant à l'absorption par le sang artériel pulsatile, et une composante continue correspondant à l'absorption par les autres tissus traversés, et des moyens de calcul agencés pour traiter les composantes variables et en extraire les taux respectifs $HbO_2$ et en déoxyhémoglobine, ou Hb, puis en déduire le taux de saturation en oxygène, dispositif caractérisé en ce que, pour tenir compte d'un taux en carboxyhémoglobine, ou HbCO, il comporte une source émettant dans une bande de longueur d'onde intermédiaire entre les deux premières bandes citées, les trois trajets optiques émanant des trois sources (11, 12, 13) émettant chacune sur une differente bande de longueur d'onde parmi les trois bandes de longueur d'onde citées et étant définis, entre la source de lumière et la région tissulaire traversée, par des fibres optiques liées en faisceau à leur extrémité proche de la région tissulaire, et aboutissant au capteur unique, des moyens aptes à exciter en succession cyclique les trois sources lumineuses, avec une fréquence de succession cyclique grande devant la fréquence du pouls du sujet, en sorte que les signaux représentatifs soient entrelacés, des moyens, commandés au rythme de la succession cyclique pour aiguiller chacun des signaux représentatifs sur une voie respective, comportant un convertisseur analogique/numérique précédant un moyen de séparation de la composante variable du signal représentatif, les moyens de calcul étant capables de traiter conjointement les trois composantes variables pour faire apparaître $HbO_2$, Hb, et HbCO, et en déduire le taux de saturation en oxygène.

Le dispositif est défini en termes d'éléments structurels aptes à exécuter les fonctions nécessaires aux opérations dont la séquence définit le procédé de l'invention.

De préférence, les sources de lumière sont constituées par des diodes lasers, sensiblement monochromatiques et capables de flux lumineux de pointe élevé.

En outre, les moyens pour définir les trajets optiques entre les sources lumineuses et les tissus seront avantageusement des fibres optiques, liées en faisceau à leur extrémité proche de la région tissulaire traversée. On comprend qu'ainsi, les trajets optiques entre fibres optiques et capteur sont sensiblement parallèles et accolés, pour traverser pratiquement les mêmes tissus sur la même longueur.

En sortie d'un microprocesseur convenablement programmé pour effectuer les calculs nécessaires, mais en soi classiques dès lors que les formules sont correctement posées, on disposera avantageusement un moyen d'enregistrement, qui enregistrera périodiquement les données du microprocesseur, pour, par lecture subséquente, rétablir les variations de saturation en oxygène, en fonction des conditions auxquelles le sujet aura été soumis.

De préférence le dispositif sera agencé en unité portable avec une autonomie d'au moins quelques heures, et mieux d'au moins 24 heures. Il est à peine nécessaire de souligner l'intérêt de pouvoir couvrir un cycle circadien complet, débutant et finissant à une même heure commode.

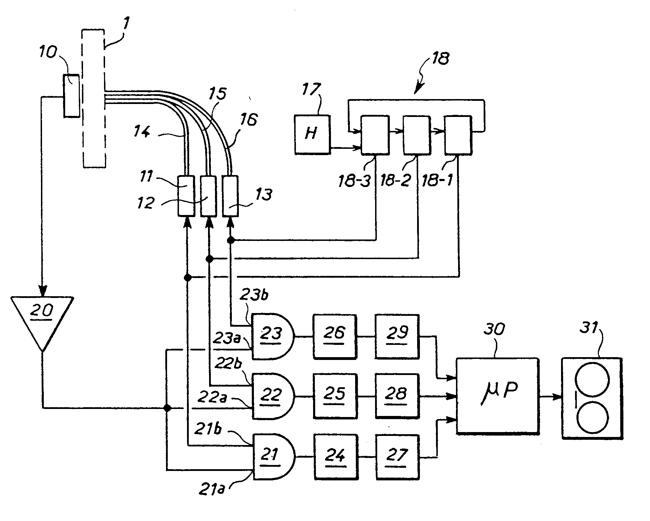

Des caractéristiques secondaires et les avantages de l'invention ressortiront d'ailleurs de la description qui va suivre à titre d'exemple, en référence au dessin annexé dans lequel la figure unique est un schéma de principe d'un dispositif selon l'invention.

Selon le mode de réalisation choisi et représenté sur cette figure, on a disposé, de part et d'autre d'une région tissulaire 1 bien vascularisée d'un sujet d'expérience, par exemple un pavillon d'oreille, trois émetteurs de lumière constitués chacun d'une diode laser 11, 12, 13, et d'une fibre optique 14, 15, 16, respectivement, les fibres optiques étant réunies et accolées en faisceau perpendiculaire à la région tissulaire 1, et un capteur opto-électronique 10. Les diodes lasers sont susceptibles d'émettre sur des longueurs d'ondes respectives de 660, 750 et 940 nm, respectivement, à quelques nanomètres près. Les trajets optiques à travers la région tissulaire 1 depuis la tranche d'extrémité des fibres optiques 14, 15 et 16 jusqu'au capteur sont donc presque confondus et perpendiculaires au plan général de la région tissulaire 1.

Le capteur opto-électronique est choisi pour avoir, sinon une sensibilité uniforme entre 660 et 940 nm, au moins une variation de sensibilité monotone et surtout sans bandes de résonance ou de coupure. Le capteur optoélectronique est suivi d'un amplificateur 20, tel que le niveau de signal utile en sortie d'amplificateur soit convenable.

Une horloge 17 attaque un compteur par trois, monté en anneau dont les sorties 18-1, 18-2 et 18-3 attaquent respectivement les diodes lasers 11, 12 et 13, de façon que celles-ci émettent, chacune à leur tour une impulsion lumineuse, sur leurs longueurs d'ondes respectives.

La fréquence de fonctionnement de l'horloge 17, triple de la fréquence d'un cycle du compteur en anneau 18 est choisie grande devant la fréquence de pulsation du sujet, qui est comprise en général entre 1 et 2 hertz, pour que les composantes variables d'absorption, à la fréquence de pulsation du sang artériel soient convenablement reproduites. Une fréquence d'horloge de quelques kilohertz conviendra bien.

On aura compris que les diodes lasers 11, 12 et 13 possèdent leurs propres circuits d'alimentation et de déclenchement en impulsion, les signaux émanant du compteur en anneau 18 étant des signaux de pilotage, et non des signaux de puissance.

Les impulsions lumineuses fournies par les diodes lasers 11, 12 et 13, et guidées par les fibres optiques 14, 15 et 16 traversent la région tissulaire 1 où elles subissent des absorptions, et parviennent au capteur 10, où elles provoquent la formation de signaux électriques fonction de l'énergie lumineuse reçue, et donc significatifs de l'absorption moléculaire subie. Ces signaux sont portés à un niveau convenable par un amplificateur 20. La succession cyclique de l'émission des trois diodes 11, 12 et 13 se traduit par un entrelacement des signaux correspondant aux trois longueurs d'onde.

En sortie de l'amplificateur 20, les signaux attaquent un aiguillage formé de trois portes ET analogiques 21, 22 et 23. Ces signaux sont appliqués en parallèle sur les entrées logiques 21a, 22a et 23a, tandis que les entrées de commande logique 21b, 22b et 23b sont attaquées par les signaux issus des sorties 18-1, 18-2 et 18-3 respectivement du compteur en anneau 18.

En sortie des portes ET 21, 22 et 23 sont disposées trois voies respectives, constituées chacune d'un convertisseur analogique/numérique 24, 25, 26, puis d'un étage séparateur de composante variable 27, 28, 29.

L'ouverture des portes 21, 22 et 23 étant respectivement synchrone des émissions des signaux lumineux par les diodes lasers 11, 12 et 13, la voie disposée en aval de la porte 21 traitera les signaux significatifs de l'absorption à 660 nm, la voie en aval de la porte 22, ceux de l'absorption à 750 nm et la voie en aval de la porte 23, ceux de l'absorption à 940 nm.

Les convertisseurs analogiques/numériques 24, 25 et 26 délivrent donc des signaux numériques représentatifs de l'absorption aux trois longueurs d'onde à des entrées d'un microprocesseur 30 après suppression de la composante continue. Celui-ci effectuera en premier lieu des calculs par voie pour traduire les signaux représentatifs d'absorption variable, numérisés, en données de densité optique, en tenant compte d'ajustements pour le rendement des diodes lasers, d'absorptions spécifiques des circuits optiques, et du rendement du capteur 10 à la longueur d'onde considérés, les paramètres d'ajustement étant déterminés par étalonnage approprié ; puis le microprocesseur résout l'équation (4), pour les inconnues Hb, $HbO_2$ et HbCO ; des valeurs de Hb, $HbO_2$ et HbCO, le microprocesseur 30 établit (équations (5) et (6)) les valeurs de $SaO_2$ et FCO.

Ces valeurs sont mises en mémoires, en accumulation. Périodiquement, les mémoires sont vidées pour déterminer les valeurs moyennes de $SaO_2$ et FCO sur la période qui s'achève. Ces valeurs sont envoyées à un enregistreur 31, par exemple du type à bande magnétique en cassette. La lecture de la cassette permettra par la suite de restituer les valeurs de $SaO_2$ et FCO avec leurs variations correspondant aux conditions subies par le sujet au cours de la période de contrôle.

Le dispositif sera normalement réalisé en deux éléments. Une sonde regroupera une monture d'extrémité pour les fibres optiques 14, 15 et 16, et le capteur 10, dans une structure à la fixation de part et d'autre de la région tissulaire choisie, par exemple le pavillon d'une oreille. L'autre élément comprendra les diodes 11, 12 et 13, les différents circuits de pilotage, d'aiguillage et de calcul, avec l'enregistreur et une alimentation autonome, du type à batterie rechargeable, et sera regroupé dans un boîtier formant sacoche. Un cordon, contenant le conducteur de liaison du capteur 10, et les fibres optiques 11, 12, 13, reliera le boîtier à la sonde. L'ensemble évoquera quelque peu un lecteur de cassette du type dit baladeur.

On notera qu'il est possible de ne loger dans le cordon qu'une fibre optique, disposée en sortie d'un multiplexeur attaqué par les fibres optiques 11, 12 et 13, raccourcies en conséquence, cette disposition permettant d'ailleurs que, dans la traversée des tissus, les trois chemins optiques soient strictement confondus.

On appréciera que la conception intégrée des calculs spécifiques de $SaO_2$ et FCO, qui remplace l'exploitation des données d'absorption dans un système de calcul banalisé suivant les méthodes actuelles, permet une miniaturisation du matériel, qui rend possible les réalisations d'un appareil autonome portable, et en corollaire l'exécution de mesures dans le cadre d'activités physiques naturelles, et sur des périodes allant jusqu'au cycle circadien.

Bien entendu, l'invention n'est pas limitée à l'exemple décrit, mais en embrasse toutes les variantes d'exécution, dans le cadre des revendications.

Notamment, tout ce qui a trait au pilotage de la succession cyclique des déclenchements des diodes lasers, et à l'aiguillage des signaux représentatifs peut être réalisé par le microprocesseur 30 convenablement programmé; l'exécution en circuits distincts décrite ci-dessus correspondait à un souci de clarté de la description.

On a déjà évoqué la concentration des trois chemins optiques des trois longueurs d'ondes en un tronçon commun comprenant la traversée de la zone tissulaire, les trois fibres optiques liées aux sources aboutissant à un multiplexeur d'où part une fibre unique.

D'un autre côté, la création d'un logiciel adapté sort du cadre de la présente invention, et est du ressort de programmeurs spécialistes dès lors qu'ils ont connaissance de la nature des données d'entrées, et du traitement qu'elles doivent subir pour aboutir aux données de sortie appropriées.

Enfin, il va de soi que l'enregistreur 31 pourra être de tout type approprié, par exemple à "disque" souple au lieu de bande magnétique à cassette.

## Revendications

1. Procédé de détermination, sur une période prolongée, du taux de saturation en oxygène du sang artériel d'un sujet, par comparaison du taux d'oxyhémoglobine, ou $HbO_2$ au taux d'hémoglobine total, où l'on acquiert, sur un capteur optoélectronique unique (10), des signaux représentatifs d'absorption optique sur des trajets traversant une région tissulaire (1) appropriée du sujet et émanant de sources lumineuses (11-13) émettant alternativement dans deux bandes de longueurs d'onde, une dans le proche infrarouge et l'autre dans le rouge profond, en sorte que ces signaux représentatifs comportent une composante variable correspondant à l'absorption par le sang artériel pulsatile, et une composante continue correspondant à l'absorption par les autres tissus, on calcule, à partir des composantes variables, les taux respectifs $HbO_2$ et en déoxyhémoglobine, ou Hb, et on en déduit le taux de saturation en oxygène, procédé caractérisé en ce que, pour tenir compte d'un taux en carboxyhémoglobine, ou HbCO, on met en oeuvre une source (12) émettant dans une bande de longueurs d'onde comprise entre les deux premières bandes citées (11-13), les trajets optiques, émanant des trois sources (11, 12, 13) émettant chacune sur une differente bande de longueur d'onde parmi les trois bandes de longueur d'onde citées, étant définis en sorte d'être, dans la région tissulaire traversée, sensiblement parallèles et accolés, et aboutissant au capteur unique (10), on excite, en succession cyclique (18), les trois sources (11, 12, 13), la fréquence de succession cyclique étant grande devant la fréquence de pulsation artérielle du sujet, en sorte que les signaux représentatifs soient entrelacés, on aiguille (21, 22, 23), au rythme de la succession cyclique, chaque signal représentatif sur une voie séparée respective (24, 27; 25, 28; 26, 29) où l'on exprime numériquement le signal représentatif, on sépare (24, 25, 26) la composante variable respective de chaque signal représentatif, et on calcule $HbO_2$, Hb et HbCO en résolvant l'équation suivante:

$$DO\lambda 1 = [e_1(Hb) + e_2(HbO_2) + e_5(HbCO)] * \underline{l}$$

$$DO\lambda 3 = [e_3(Hb) + e_4(HbO_2) + e_6(HbCO)] * \underline{l}$$

$$DO\lambda 2 = [e_7(Hb) + e_8(HbO_2) + e_9(HbCO)] * \underline{l}$$

où $DO\lambda_n$ représente la composante variable d'absorption à la longueur d'onde $\lambda_n$, $\underline{l}$ est la profondeur de tissu traversé, et $e_j$ un coefficient d'absorption spécifique de la longueur d'onde et de l'espèce d'hémoglobine déterminé expérimentalement;

et l'on en déduit le taux de saturation en oxygène.

2. Procédé suivant la revendication 1, caractérisé en ce que les bandes de longueurs d'onde sont centrées sur 940 nm, 660 nm et 750 nm, respectivement.

3. Procédé suivant l'une des revendications 1 et 2, caractérisé en ce que le traitement des signaux représentatifs d'absorption comprenant l'extraction (24, 25, 26) des composantes variables et les calculs subséquents sont effectués dans un microprocesseur (32) sous la commande d'un logiciel approprié.

4. Dispositif pour la détermination, sur une période prolongée, du taux de saturation en oxygène du sang artériel d'un sujet, par comparaison du taux en oxyhémoglobine, ou $HbO_2$, au taux d'hémoglobine total, comportant des moyens aptes à définir des trajets optiques traversant une région tissulaire (1) appropriée du sujet entre des sources lumineuses (11, 13) émettant alternativement dans deux bandes de longueurs d'onde différentes situées respectivement dans l'infrarouge proche et dans le rouge profond, et un capteur optoélectronique unique (10) apte à émettre des signaux représentatifs de l'absorption optique sur les trajets et comportant une composante variable correspondant à l'absorption par le sang artériel pulsatile, et une composante continue correspondant à l'absorption par les autres tissus traversés, et des moyens de calcul (30) agencés pour traiter les composantes variables et en extraire les taux respectifs $HbO_2$ et en déoxyhémoglobine, ou Hb, puis en déduire le taux de saturation en oxygène, dispositif caractérisé en ce que, pour tenir compte d'un taux en carboxyhémoglobine, ou HbCO, il comporte une source (12) émettant dans une bande de longueur d'onde intermédiaire entre les deux premières bandes citées, les trois trajets optiques émanant des trois sources (11, 12, 13) émettant chacune sur une differente bande de longueur d'onde parmi les trois bandes de longueur d'onde citées et étant définis, entre la source de lumière (11, 12, 13) et la région tissulaire traversée (1), par des fibres optiques (14, 15, 16) liées en faisceau à leur extrémité proche de la région tissulaire (1), et aboutissant au capteur unique (10), des moyens (17, 18, 19) aptes à exciter en succession cyclique les trois sources lumineuses (11, 12, 13), avec une fréquence de succession cyclique grande devant la fréquence du pouls du sujet, en sorte que les signaux représentatifs soient entrelacés, des moyens (21, 22, 23), commandés au rythme de la succession cyclique pour aiguiller chacun des signaux représentatifs sur une voie respective (24, 27; 25, 28; 26, 29), comportant un convertisseur analogique/numérique (24, 25, 26) précédant un moyen de séparation (27, 28, 29) de la composante variable du signal représentatif, les moyens de calcul (30) étant capables de traiter conjointement les trois composantes variables pour faire apparaître $HbO_2$, Hb, et HbCO, et en déduire le taux de saturation en oxygène.

5. Dispositif selon la revendication 4, caractérisé en ce que les sources de lumière sont constituées par des diodes lasers (11, 12, 13).

6. Dispositif selon l'une quelconque des revendications 4 et 5, caractérisé en ce qu'il comporte un microprocesseur (30) programmé en sorte d'exécuter les calculs d'extraction des $HbO_2$, Hb et HbCO.

7. Dispositif selon la revendication 6, caractérisé en ce qu'il comporte un moyen d'enregistrement (31) apte à enregistrer les données issues du microprocesseur périodiquement.

8. Dispositif selon la revendication 7, caractérisé en ce qu'il est agencé en unité portable avec une alimentation autonome de capacité telle qu'elle confère au dispositif une autonomie d'au moins quelques heures.

9. Dispositif selon la revendication 8, caractérisé en ce que la capacité de l'alimentation autonome est telle qu'elle confère au dispositif une autonomie d'au moins 24 heures.

**Claims**

1. Method for determining, over a prolonged period, the oxygen saturation level of the arterial blood of a subject, by comparing the level of oxyhaemoglobin, or $HbO_2$, with the total haemoglobin level, in which there are acquired, on a optoelectronic sensor (10), signals representing optical absorption on paths passing through an appropriate tissue area (1) in the subject and emanating from light sources (11-13), emitting alternately in two wavelength bands, one in the near infrared and the other in the deep red, so that these representative signals have a variable component corresponding to the absorption by the pulsatile arterial blood, and a continuous component corresponding to the absorption by the other tissues, the respective levels of $HbO_2$ and deoxyhaemoglobin, or Hb, are calculated on the basis of the variable components, and the oxygen saturation level is derived therefrom, a method characterised in that, in order to take account of a level of carboxyhaemoglobin, or HbCO, a source (12) emitting in a wavelength band between the aforementioned first two bands (11-13) is used, the optical paths, emanating from the three sources (11, 12, 13) each emitting in a different wavelength band amongst the aforementioned three wavelength bands, being defined so as to be, in the tissue area passed through, substantially parallel and side by side, and ending in the single sensor (10), the three sources (11, 12, 13) are excited in cyclical succession (18), the cyclical succession frequency being high in comparison with the arterial pulsation frequency of the subject, so that the representative signals are interlaced, and each representative signal is switched (21, 22, 23), at the rate of the cyclic succession, onto a respective separate channel (24, 27; 25, 28; 26, 29) in which the representative signal is expressed numerically, the respective variable component of each representative signal is separated (24, 25, 26) and $HbO_2$, Hb and HbCO are calculated by resolving the following equation:

$$DO\lambda 1 = [e_1(Hb) + e_2(HbO_2) + e_5(HbCO)] * \underline{l}$$

$$DO\lambda 3 = [e_3(Hb) + e_4(HbO_2) + e_6(HbCO)] * \underline{l}$$

$$DO\lambda 2 = [e_7(Hb) + e_8(HbO_2) + e_9(HbCO)] * \underline{l}$$

where $DO\lambda_n$ represents the variable absorption component at the wavelength $\lambda_n$, $\underline{l}$ is the depth of tissue passed through and $e_j$ a specific absorption coefficient of the wavelength and haemoglobin type determined experimentally; and the oxygen saturation level is derived therefrom.

2. Method according to claim 1, characterised in that the wavelength bands are centred on 940 nm, 660 nm and 750 nm respectively.

3. Method according to one of Claims 1 and 2, characterised in that the processing of the representative absorption signals, comprising the extraction (24, 25, 26) of the variable components, and the subsequent calculations, are effected in a microprocessor (32) under the control of a suitable software program.

4. Device for determining, over a prolonged period, the oxygen saturation level of the arterial blood of a subject, by comparing the level of oxyhaemoglobin, or $HbO_2$, with the total haemoglobin level, including means suitable for defining optical paths passing through an appropriate tissue area (1) in the subject between light sources (11, 13), emitting alternately in two wavelength bands with different wavelengths situated, respectively, in the near infrared and in the deep red, and a single optoelectronic sensor (10) suitable for emitting signals representing the optical absorption on the paths and having a variable component corresponding to the absorption by the pulsatile arterial blood, and a continuous component corresponding to the absorption by the other tissues passed through, and calculation means (30) arranged so as to process the variable components and extract the respective $HbO_2$ and deoxyhaemoglobin, or Hb, levels therefrom, and then derive the oxygen saturation level therefrom, a device characterised in that, in order to take account of a level of carboxyhaemoglobin, or HbCO, it has a source (12) emitting in a wavelength band intermediate between the aforementioned first two bands, the three optical paths emanating from the three sources (11, 12, 13) each emitting in a different wavelength band amongst the aforementioned three wavelength bands, and being defined, between the light source (11, 12, 13) and the tissue area (1) passed through, by optical fibres (14, 15, 16) connected in a bundle at their end close to the tissue area (1) and ending at the single sensor (10), means (17, 18, 19) suitable for exciting the three light sources (11, 12, 13) in cyclical succession, with

EP 0 524 083 B1

a high cyclical succession frequency in comparison with the frequency of the pulse of the subject, so that the representative signals are interlaced, means (21, 22, 23), controlled at the rate of the cyclical succession, for switching each of the representative signals onto a respective channel (24, 27; 25, 28; 26, 29), having an analogue to digital converter (24, 25, 26) preceding a means (27, 28, 29) for separating the variable component of the representative signal, the calculation means (30) being capable of processing conjointly the three variable components, to identify $HbO_2$, Hb and HbCO, and derive the oxygen saturation level therefrom.

5. Device according to Claim 4, characterised in that the light sources consist of laser diodes (11, 12, 13).

6. Device according to either one of Claims 4 and 5, characterised in that it includes a microprocessor (30) programmed so as to effect the $HbO_2$, Hb and HbCO extraction calculations.

7. Device according to Claim 6, characterised in that it has a recording means (31), suitable for recording the data coming from the microprocessor periodically.

8. Device according to Claim 7, characterised in that it is arranged in a portable unit with an autonomous power supply with a capacity such that it confers at least a few hours of autonomous operation on the device.

9. Device according to Claim 8, characterised in that the autonomous power supply is such that it confers at least 24 hours of autonomous operation on the device.

**Patentansprüche**

1. Verfahren zur Bestimmung des arteriellen Blutsauerstoffsättigungsgehaltes eines Patienten über einen längeren Zeitraum hinweg durch Vergleich des Oxyhämoglobin- bzw. $HbO_2$-Gehalts mit dem Gesamthämoglobingehalt, indem man an einem einzigen optoelektronischen Meßfühler (10) repräsentative optische Absorptionssignale auf Pfaden erhält, die einen geeigneten Gewebebereich (1) des Patienten durchqueren und abwechselnd von zwei Wellenlängenbereichen, einerseits im nahen Infrarot-, andererseits im tiefroten Bereich emittierenden Lichtquellen (11-13) ausgehen, so daß diese repräsentativen Signale sowohl eine variable Komponente, die der Absorption durch das arterielle pulsierende Blut entspricht als auch eine kontinuierliche Komponente enthalten, die der Absorption durch die anderen Gewebe entspricht, wobei man ausgehend von der variablen Komponente den $HbO_2$-und Desoxyhämoglobin- bzw. Hb-Gehalt errechnet und hieraus den Sauerstoffsättigungsgehalt ableitet, wobei dieses Verfahren dadurch gekennzeichnet ist, daß zur Berücksichtigung des Kohlenmonooxydhämoglobin- bzw. HbCO-Gehalts eine Quelle (12) eingeschaltet wird,die in einem zwischen den erstgenannten Wellenlängenbereichen (11-13) gelegenen Bereich emittiert, wobei die optischen Pfade, die von den drei Lichtquellen (11, 12, 13) ausgehen, welche jeweils in verschiedenen Wellenlängen innerhalb der genannten drei Bereiche emittieren, derart definiert sind, daß sie in dem durchquerten Gewebebereich deutlich parallel aneinander anstoßend verlaufen und in dem Meßfühler (10) enden, wobei die drei Lichtquellen (11, 12, 13) in einer zyklischen Folge (18) erregt werden, wobei die zyklische Folgenfrequenz im Vergleich zur arteriellen Pulsfrequenz des Patienten groß ist, so daß die repräsentativen Signale korreliert sind, wobei im Rhythmus der zyklischen Folgenfrequenz jedes repräsentative Signal dargestellt wird (21, 22, 23) und zwar jedes repräsentative Signal auf einen jeweiligen separaten Kanal (24, 27; 25, 28; 26, 29), wobei das repräsentative Signal numerisch ausgedrückt wird, wobei die variablen Komponenten jedes repräsentativen Signals getrennt (24, 25, 26) werden, wobei $HbO_2$, Hb und HbCO mittels Lösung folgender Gleichungen berechnet werden:

$$DO\lambda 1 = [e_1 (Hb) + e_2 (HbO_2) + e_5 (HbCO)] \, {}^*\underline{1}$$

$$DO\lambda 3 = [e_3 (Hb) + e_4 (HbO_2) + e_6 (HbCO)] \, {}^*\underline{1}$$

$$DO\lambda 2 = [e_7 (Hb) + e_8 (HbO_2) + e_9 (HbCO)] \, {}^*\underline{1}$$

in deren $DO\lambda n$ die variable Absorptionskomponente bei einer Wellenlänge von $\lambda n$, $\underline{1}$ die Tiefe des durchquerten Gewebes und $e_j$ einen spezifischen Absorptionskoeffizienten der Wellenlänge und des experimentell bestimmten Hämoglobintyps darstellen; wobei der Sauerstoffsättigungsgehalt hieraus erhalten wird.

2. Verfahren gemäß Anspruch 1, dadurch gekennzeichnet, daß die Wellenlängenbereiche auf 940 nm, 660 nm bzw. 750 nm zentriert sind.

3. Verfahren gemäß Anspruch 1 oder 2, dadurch gekennzeichnet, daß die Verarbeitung der die Absorption betreffenden, repräsentativen Signale, die Extraktion (24, 25, 26) der variablen Komponenten, wobei die folgenden Berechnungen, in einem mit einer entsprechenden Software augestatteten Mikroprozessor (32) erfolgen, umfaßt.

4. Vorrichtung für die Bestimmung des arteriellen Blutsauerstoffsättigungsgehaltes eines Patienten über einen längeren Zeitraum hinweg durch Vergleich des Oxyhämoglobin- bzw. $HbO_2$-Gehalts mit dem Gesamthämoglobingehalt, umfassend Mittel, die in der Lage sind, optische Pfade zu definieren, die einen geeigneten Gewebebereich (1) des Patienten durchqueren, und zwar zwischen den Lichtquellen (11, 13), welche wechselweise in zwei unterschiedlichen Wellenlängenbereichen emittieren, einerseits im nahen Infrarot, andererseits im tiefroten Bereich, und einen einzigen optroelektronischen Meßfühler (10), welcher in der Lage ist, repräsentative Signale bezüglich der optischen Absorption auf den Pfaden auszusenden, umfassend sowohl eine variable Komponente, die der Absorption durch das pulsierende arterielle Blut entspricht, als auch eine kontinuierliche Komponente, die der Absorption durch die anderen durchquerten Gewebe entspricht; und ein Rechenwerk (30), das so ausgelegt ist, daß es die variablen Komponenten verarbeiten kann und davon die jeweiligen $HbO_2$- und die Deoxyhämoglobin- bzw. Hb-Gehalte extrahieren und hieraus den Sauerstoffsättigungsgehalt ableiten kann, wobei die Vorrichtung dadurch gekennzeichnet ist, daß sie zur Berücksichtigung des Kohlenmonooxydhämoglobin- bzw. HbCO-Gehalts umfaßt: eine Lichtquelle (12), die in einem zwischen den beiden erstgenannten Wellenlängenbereichen gelegenen Bereich emittiert, wobei die drei optischen Pfade, die von den drei Lichtquellen (11, 12, 13) ausgehen, welche jeweils in verschiedenen Wellenlängen innerhalb der genannten drei Bereiche emittieren, zwischen den Lichtquellen (11, 12, 13) und dem durchquerten Gewebebereich (1) definiert sind, und zwar durch optische Fasern (14, 15, 16), die an ihrem gewebeseitigen Ende gebündelt sind und am einzigen Meßfühler (10) enden; Einrichtungen (17, 18, 19), die eine Erregung der drei Lichtquellen (11, 12, 13) in zyklischer Reihenfolge bewirken können, wobei die zyklische Folgenfrequenz im Vergleich zur Pulsfrequenz des Patienten groß ist, so daß die repräsentativen Signale korreliert sind; Einrichtungen (21, 22, 23), die im Rhythmus der zyklischen Folge gesteuert werden, damit jedes einzelne der repräsentativen Signale auf einen jeweiligen Kanal (24, 27; 25, 28; 26, 29) dargestellt wird, umfassend einen Analog-Digital-Wandler (24, 25, 26), der einer Trennvorrichtung (27, 28, 29) für die variable Komponente des repräsentativen Signals vorgeschaltet ist; ein Rechenwerk (30), das in der Lage ist, die drei variablen Komponenten zusammen zu verarbeiten, so daß die $HbO_2$-, Hb- und HbCO-Gehalte angezeigt werden und daraus der Sauerstoffsättigungsgehalt abgeleitet werden kann.

5. Vorrichtung gemäß Anspruch 4, dadurch gekennzeichnet, daß die Lichtquellen aus Laserdioden (11, 12, 13) gebildet sind.

6. Vorrichtung gemäß Anspruch 4 oder 5, dadurch gekennzeichnet, daß diese einen Mikroprozessor (30) enthält, der so programmiert ist, daß er die Berechnungen zum Extrahieren des $HbO_2$, Hb und HbCO durchführen kann.

7. Vorrichtung gemäß Anspruch 6, dadurch gekennzeichnet, daß diese ein Datenerfassungsgerät (31) enthält, das die vom Mikroprozessor ausgegebenen Daten in regelmäßigen Abständen speichert.

8. Vorrichtung gemäß Anspruch 7, dadurch gekennzeichnet, daß diese als tragbares Gerät mit einer autonomen Stromversorgung ausgelegt ist, die eine solche Kapazität aufweist, daß sie der Vorrichtung eine Autonomie von mindestens einigen Stunden verleiht.

9. Vorrichtung gemäß Anspruch 8, dadurch gekennzeichnet, daß die Kapazität der Autonomieversorgung derart ist, daß sie der Vorrichtung eine Autonomie von 24 Stunden verleiht.